# EUROPÄISCHE PATENTSCHRIFT

(11) **EP 0 612 252 B1**
(45) Veröffentlichungstag und Bekanntmachung des Hinweises auf die Patenterteilung: **12.05.1999**
(21) Anmeldenummer: 93919123.5
(22) Anmeldetag: 20.08.1993
(51) Int. Cl.: A61L 15/32, C08H 1/06

(54) **WUNDABDECKUNGSMATERIAL AUF BASIS VON KOLLAGENFASERN**
WOUND COVERING MATERIAL BASED ON COLLAGEN FIBRES
MATERIAU DE RECOUVREMENT DE PLAIES A BASE DE FIBRES DE COLLAGENE

(30) Priorität: 21.08.1992 DE 4227681
(43) Veröffentlichungstag der Anmeldung: 31.08.1994
(73) Patentinhaber: NATURIN GmbH & CO., D-69448 Weinheim (DE)
(72) Erfinder: MASER, Franz, D-68159 Mannheim (DE)
(74) Vertreter: Siewers, Gescha, Dr.
(86) Internationale Anmeldenummer: EP9302239
(87) Internationale Veröffentlichungsnummer: WO9404201

(56) Entgegenhaltungen:
- EP-A- 0 331 786
- DE-A- 2 734 503
- DE-A- 2 943 520
- GB-A- 2 079 797

## Beschreibung

Die Erfindung betrifft Wundabdeckungsmaterial in Folienform auf Basis von Kollagenfasern.

Bei zahlreichen Verletzungen, aber auch bei medizinischen Eingriffen sind oftmals Hauttransplantationen notwendig, für die aber nicht immer geeignete Eigen- oder Spenderhaut zur Verfügung steht. Seit langem versucht man daher, besonders großflächige Wunden wie Brandwunden mit anderen Materialien abzudecken, um Flüssigkeitsverluste und externe Infektionen zu verhindern. Seit einigen Jahren wird auf diesem Gebiet auch mit "Kunsthaut" auf Basis von Kollagenfasern experimentiert. Die bisherigen Ergebnisse mit einer solchen Art "Kunsthaut" werden unterschiedlich beurteilt.

Als Kollagen bezeichnet man die langfaserigen, linearcolloiden hochmolekularen Skleroproteine der extrazellulären Matrix, die im Bindegewebe, in der eiweißhaltigen Grundsubstanz des Knochens und im Dentin zusammen mit Proteoglykanen vorkommen. Die Zusammensetzung der Kollagene kann je nach Herkunft variieren, man kennt verschiedene Typen der Kollagene, die jedoch nicht alle eine Faserstruktur besitzen. Auffällig bei den Kollagenen ist der geringe Anteil an Tryptophan, Tyrosin und Cystin, dagegen zeichnen sich Kollagene durch einen hohen Anteil an Glycin, Prolin und insbesondere an 4-Hydroxyprolin aus. Kollagene werden in Fibroblasten, also in Bindegewebszellen zunächst als Prokollagenketten mit einem Molekulargewicht von etwa 140.000 synthetisiert. Erst in der Kette erfolgt die Hydroxilierung von Prolin und Lysin unter Einwirkung von Ascorbinsäure und die Glykosilierung, woran anschließend jeweils 3 Ketten zusammentreten und zwar in Form linksgewundener Helices, die wiederum rechtsgängig umeinander gedreht sind. Diese Substanz wird dann in den extrazellulären Raum ausgeschieden, wo von den Enden der Ketten Peptide abgespalten werden, so daß das sogenannte Tropokollagen entsteht, daß sich zu Fibrillen zusammenlagert. Während das Tropokollagen noch salz- bzw. säurelöslich ist, sind Kollagenfibrillen unlöslich. Das Tropokollagen mit einem Molekulargewicht von etwa 300.000 besteht aus 3 Polypeptidketten, die eine etwas unterschiedliche Aminosäuresequenz aufweisen können. In der Regel sind 2 Ketten identisch, die dritte weist einen abweichenden Aufbau auf.

Im Gegensatz zu den meisten Proteinen des menschlichen oder tierischen Körpers werden die Kollagene nicht laufend erneuert, sondern haben eine lange biologische Halbwertzeit, die bis zu 300 Tagen betragen kann. Gegen enzymatische Zersetzung sind die Kollagene recht beständig; ein enzymatischer Abbau nativen Kollagens läßt sich im wesentlichen nur durch Kollagenase erreichen. Die nach Proteolyse der Kollagenfabrillen entstehenden löslichen Spaltprodukte werden durch andere Proteasen zu Peptiden und Aminosäuren hydrolisiert.

Sowohl lösliche als auch unlösliche Kollagene sind bereits als "Kunsthaut" experimentell eingesetzt worden. Entsprechende Produkte sind beispielsweise in der japanischen Patentanmeldung 59 160464 oder im U.S.-Patent 46 00 533 beschrieben. Auch für Endoprothesen ist das Material bereits vorgeschlagen worden, so beispielsweise in der europäischen Patentanmeldung 85 20 0045. Eine derartige "Kunsthaut" aus Kollagen vermindert den Flüssigkeitsverlust und schützt gegen externe Infektionen, außerdem soll diese Art Kunsthaut eine entzündungshemmende und haemostatische Wirkung haben und das Wachstum von Epithelzellen beschleunigen. Die bisher als Rohstoff eingesetzten Kollagene sind in der Regel aufgrund ihrer besseren Verarbeitbarkeit lösliche Kollagene. Dies bedingt den Nachteil, daß die Membranen oder Filme relativ nur eine geringe Festigkeit aufweisen, da sie aus nichtfaserigen Kollagenen bestehen, die in dieser Form nativ nicht vorkommen. Außerdem wird das verwendete Kollagen weiterhin enzymatisch aufgelöst. Bisher werden diese Membranen oder Filme aus Kollagengelen hergestellt, die nach unterschiedlichen an und für sich bekannten Techniken erzeugt und dann in eine feste Form überführt werden. Entsprechende Produkte sind beispielsweise in den U.S.-Patenten 4600533, 4689399 oder 4725641 und 4294241 beschrieben. Die mangelnde Haltbarkeit solcher Filme versucht man entsprechend U.S.-Patent 3800792 dadurch zu verbessern, daß die aus einem Kollagengel hergestellte Schwammstruktur vernetzt und mit einem plastischen Film versehen wird, wodurch aber die Anpassung und Adhärenz des Materials an die Wunde deutlich verschlechtert wird. Nachteilig bei allen bisher bekannten Kollagenfilmen als Verbandmaterial ist die Tatsache, daß wie bereits dargelegt, die Adhärenz zur Wunde bei diesen Filmen nicht sehr ausgeprägt ist, so daß in der Regel eine zusätzliche Randfixierung notwendig ist, insbesondere, wenn größere Flächen abgedeckt werden müssen. Eine weitere Schwierigkeit mit dem bisher bekannten Materialien besteht darin, daß sie relativ schnell abbaubar sind und daß sie aufgrund ihrer Aufbereitung noch einen beträchtlichen Gehalt an Proteoglykanen aufweisen, die zu einer in der Regel zwar geringen, aber in Einzelfällen deutlich ausgeprägten allergischen Reaktion führen können, da es sich trotz allem um körperfremdes, nicht humanes Material handelt. Außerdem ist es nicht ganz einfach, beispielsweise aus löslichen Kollagenen hergestellte Produkte steril zu erhalten und lagerfähig zu machen, da der Abbau bzw. der weitere Abbau der Aminosäureketten nicht völlig unterbrochen werden kann. Bei der Herstellung von Filmen oder Membranen aus unlöslichen Kollagen besteht aber stets die Schwierigkeit, die Fasern überhaupt soweit von Begleitmaterial wie beispielsweise den allergisierenden Proteoglykanen zu reinigen, daß eine unbedenkliche Verwendung, insbesondere auf größeren Körperflächen möglich ist. Aus der japanischen Patentanmeldung 61 041 462 ist bereits die Kombination eines Kollagenfilmes aus löslichem Kollagen mit Hyaluronsäure bekannt. Hyaluronsäure besteht aus den Grundbausteinen D-Glukuronsäure und N-Acetyl-D-glucosamin in 1,3-glykosidischer Bindung und kann je nach Herkunft und Aufarbeitung Molekulargewichte zwischen etwa 50.000 und mehreren Millionen aufweisen. Hyaluronsäure kann sich an Fibrine binden und bildet mit diesen eine dreidimensionale Matrix, wodurch die ursprüngliche Fibrinmatrix verformt wird, aufquillt und porös wird. Dadurch wird eine schnellere und bessere Infiltration und Migration von Zellen in die Matrix ermöglicht. Hyaluronsäure hat einen intensiven, stimulierenden Effekt auf die Geschwindigkeit der Bildung von Fibrinmatrizes, die durch Thrombin induziert wurden. So steigt kurz nach einer Verletzung der Gehalt an Hyaluronsäure in der Wunde an, wobei die Substanz außer Zellinfiltration und Quellung der Matrix, auch die Phagocytose und die Vascularisation neugebildeten Gewebes beeinflußt. Hyaluronsäure spielt daher beim Auf-, Um- und Abbau der Fibrinmatrix eine ganz wesentliche Rolle, so daß der Zusatz zu Kollagenfilmen wünschenswert ist, da Hyaluronsäure für eine schnellere und komplikationslosere Abheilung der Wunde sorgt.

Die bisher bekannten "Kunsthäute auf Kollagenbasis" weisen aber immer noch die Nachteile auf, daß sie keine gute Adhärenz zeigen, Immunreaktionen und Allergien auslösen können und in der Herstellung nicht besonders preisgünstig sind. Zudem ist die Wasserdurchlässigkeit hoch und die Abbaubarkeit zu schnell. Es besteht daher noch ein Bedürfnis nach weiteren Wundabdeckungsmaterialien auf Basis von Kollagenfasern, die diese Nachteile nicht aufweisen.

Erfindungsgemäß wird nunmehr ein Wundabdeckungsmaterial in Folienform auf Basis von Kollagenfasern vorgeschlagen, das dadurch gekennzeichnet ist, daß es aus unlöslichem, teilmodifizierten Kollagen besteht, das sich insbesondere durch folgende Parameter vom nativen Kollagen unterscheidet, nämlich hinsichtlich Amidstickstoff, Glucosamin- und Galactosamingehalt, isoelektrischer Punkt und Schrumpfungstemperatur. überraschenderweise wurde jetzt festgestellt, daß durch die Teilmodifizierung von nativem, unlöslichen Kollagen eine Substanz gewonnen werden kann, die sich hervorragend zur Herstellung von als Verbandmaterial einsetzbaren Filmen eignet. Die Modifizierung erfolgt durch eine schonende Hydrolyse, wobei insbesondere der Anteil der Amidgruppen des Asparagins und des Glutamins reduziert und damit der isoelektrische Punkt verschoben werden. Außerdem erfolgt eine Spaltung von Quervernetzungen der natürlichen Fibrillen, aber im wesentlichen bleibt die Fibrillenstruktur des nativen unlöslichen Kollagens erhalten. Die schonende Hydrolyse kann in verschiedener, dem Fachmann auf dem Proteinsektor bekannte Weise durchgeführt werden. Als Rohstoff werden erfindungsgemäß Rinderhautspalten nach dem den Fachleuten geläufigen Aufbereitungsverfahren, beispielsweise gemäß der DE-PS 659 490 sauer gequollen. Der Trockenstoffgehalt der so vorbehandelten Kollagenschwarten beträgt etwa 16,0 Gew.-%, der Amidstickstoffgehalt liegt bei 0,28 mmol/gr Trockenmasse, der Aschegehalt beläuft sich auf etwa 0,4 Gew.-% (bezogen auf Trockenmasse) und er pH-Wert beträgt 2,8 bis 2,9. Diese vor vorbehandelten Schwarten werden dann in geeigneter Weise vorzerkleinert. Die Schwartenmasse wird dann mit Eis und Wasser aufgenommen und diese Suspension wird zusätzlich mit Glycerin und Sorbit oder sonstigen physiologisch unbedenklichen Feuchthaltemitteln versetzt. Ggf. kann auf dieser Verarbeitungsstufe zusätzlich ein physiologisch unbedenklicher organischer oder anorganischer Gerbstoff zugesetzt werden. Die Suspension wird im Ansatzbehälter aufgerührt und sorgfältig vermischt; der pH-Wert wird mit Salzsäure wieder auf etwa 2,8 bis 2,9 eingestellt. Die Partikel dieser Suspension werden dann in an sich bekannter Weise weiter zerkleinert, so daß schließlich eine makroskopisch homogen erscheinende, gelartig gequollene Dispersion mit einem pH-Wert von etwa 2,8 und einem Trockenkollagengehalt von 2,0 % erhalten wird. Diese Dispersion wird anschließend als Folie in an sich bekannter Weise extrudiert. Die noch feuchte Folie wird neutralisiert auf einen physiologisch annehmbaren Bereich, vorzugsweise pH 5, beispielsweise durch Begasung mit Ammoniak.Vor dem Wickeln wird die Folie konditioniert durch Wiederanheben des Wassergehaltes auf etwa 12 bis 18 und vorzugsweise etwa 15 Gewichtsprozent und kann dann verpackt und gelagert bzw. verwendet werden. Das so hergestellte Material ist sterilisierbar und, soweit die bisherigen Untersuchungen ergeben haben, fast unbegrenzt lagerfähig, da chemische Reaktionen auch bei längerer Lagerung praktisch nicht ablaufen.

Wenn die Folie mit Hyaluronsäure kombiniert werden soll, wird diese in der geeigneten Konzentration entweder schon bei der Herstellung der Suspension zugegeben oder nachträglich nach der Extrusion auf den fertigen Film aufgeschichtet. Anstelle von Hyaluronsäure können aber auch andere Substanzen zugegeben werden, die die Wundheilung positiv beeinflussen wie beispielsweise Antibiotika, zellwachstumsfördernde Substanzen, blutstillende Verbindungen oder Adhäsionsfaktoren. Die Einarbeitung erfolgt wie im Falle der Hyaluronsäure entweder bei der Herstellung der Suspension oder durch nachträgliche Aufbringung auf die extrudierte Folie.

Das erfindungsgemäße Wundabdeckungsmaterial zeichnet sich dadurch aus, daß im Vergleich zum Ausgangsmaterial Amidstickstoff, Glucosamin- und Galactosamingehalt, isoelektrischer Punkt und Schrumpfungstemperatur deutlich verändert sind. Im Durchschnitt ergeben sich beim Ausgangsmaterial der frischen Rinderhaut folgende Werte:
Schrumpfungstemperatur 68°C
Amidstickstoff 0,52 mmol/g
Isoelektrischer Punkt 7,0 bis 7,8

Im Vergleich dazu weist das erfindungsgemäß modifizierte Basiskollagen folgende Werte auf:
Schrumpfungstemperatur 45-60°C
Amidstickstoff 0,18-0,40 mmol/g
Isoelektrischer Punkt 4,3 bis 6,0
Glucosamin- und Gelactosamin: je kleiner als 6 µmol/g

Die erfindungsgemäßen Kollagenfolien sind deutlich reißfester als die bisher nach dem Stand der Technik hergestellten Kollagenmenbranen und sie verfügen über eine bisher unerreichte Adhärenz, da sie in feuchtem Zustand besonders elastisch und anpassungsfähig sind, wodurch eine gute Bedeckung auch bei unregelmäßigen oder unebenen Wunden ermöglicht wird und regelmäßig keine Randfixierung mehr notwendig ist. Falls erforderlich, können auch mehrere Schichten übereinandergelegt werden, ohne daß die Adhärenz negativ beeinflußt wird. Die Folien sind hydrophil, so daß Feuchtigkeit und Gase durchtreten und von der Wunde abgeleitet werden können, während das Material gleichzeitig einen Schutz gegen exogene Mikroorganismen bildet. Die Folien weisen hämostatische und schmerzstillende Eigenschaften auf und lassen sich leicht und ohne Schmerzen lösen, so daß bei Wundkontrollen oder Verbandswechsel keine unnötige Beeinträchtigung des Patienten entsteht. Besonders wichtig ist aber auch, daß die erfindungsgemäßen Folien praktisch keine allergisierende Wirkung haben, so daß selbst bei empfindlichen oder prädisponierten Patienten nicht mit Reaktionen zu rechnen ist. Das Material wird im übrigen vollständig resorbiert und im Körper abgebaut, ohne daß auch in diesem Bereich irgendwelche immunologischen Probleme selbst in schwierigen Fällen festzustellen waren.

Ein weiterer Vorzug liegt darin, daß das Ausgangsmaterial in großen Mengen hergestellt werden kann und daß die Produktionskosten für die Folie im Vergleich zu den bisher üblichen Verfahren relativ niedrig sind, so daß die Kosten eine umfangreichere Verwendung als bisher ermöglichen.

Im übrigen hat sich herausgestellt, daß es für spezielle Anwendungszwecke durchaus wünschenswert sein kann, die erfindungsgemäße Folie in Form eines Laminates einzusetzen. Die Kollagenfolie kann dabei entweder mit okklusiven Filmen auf Basis von beispielsweise Polyestern, Polymethylmetacrylaten, Polyurethanen oder Plyetherurethanen laminiert sein oder mit Filmen geschäumter Materialien wie beispielsweise Polyurethanschaum oder schwammartige Gebilde beispielsweise auf Basis von Kallagen oder Hydrokolloiden. Auch Laminate mit textilen Flächengebilden wie beispielsweise Gazen, Vliesmaterialien unterschiedlichsten Typs oder Mullbinden können in besonderen Anwendungsfällen von großem Nutzen sein. Die Herstellung solcher Laminate durch Kalandrieren, Verkleben mit physiologisch unbedenklichen Klebstoffen oder in anderer Weise ist dem Fachmann geläufig. Es ist auch möglich, die erfindungsgemäßen Folien zu matallisieren, indem in an sich bekannter Weise dünne Aluminium-, Kupfer- oder Silberschichten aufgebracht werden. Die Technik der Metallisierung von Folien ist dem Fachmann geläufig. Derartige metallisierte Folien zeichnen sich durch eine äußerst geringe Wasserdampfdurchlässigkeit aus, die in machen Fällen erwünscht ist. Bei Verwendung von oligodynamisch wirkenden Matallen wie Kupfer oder Silber kann außerdem ein bakterizider Effekt erzielt werden.

Im folgenden wird die Erfindung anhand der Beispiele näher erläutert:

### Beispiels 1

Aus Rinderhautspalten werden sauer gequollene Kollagenschwarten nach einem dem Fachmann geläufigen Aufbereitungsverfahren, beispielsweise entsprechend der DE-PS 659 490 hergestellt. Der Trockenmassegehalt beträgt 10,0 Gew.-%, der Amidstickstoffgehalt liegt bei 0,28 mol/g Trockenmasse, der Aschegehalt beläuft sich auf 0,4 Gew.-% (bezogen auf Trockenmasse) und der pH beträgt 2,0.

Diese Kollagenschwarten werden dann in einer dem Fachmann geläufigen Art grob vorzerkleinert.

45 kg der resultierenden Rohmasse werden in einem entsprechenden Ansatzbehälter mit 80 kg Eis, 212,5 l Wasser, 1,8 l Glycerin (84 gewichtsprozentig), 0,4 l Sorbit (70 gewichtsprozentig) und 0,2 kg AL₂(SO₄)₃x18H₂O versetzt. Alle Komponenten werden im Ansatzbehälter aufgerührt und sorgfältig vermischt. Der pH-Wert wird mit Salzsäure auf 2,8 eingestellt.

Ausgehend von dieser Mischung wird eine makroskopisch homogen erscheinende, entlüftete, gelartig gequollene Dispersion mit einem pH-Wert von 2,8 und einem Trockenkollagengehalt von 2,0% erzeugt, die anschließend über eine Schlitzdüse auf ein endloses Transportband extrudiert wird.

Das Transportband durchläuft einen Trockentunnel. Vor Abschluß des Trocknungsvorgangs wird die Folie mit Ammoniak begast, bis der pH-Wert der fertigen Folie bei ca. 5 liegt. Nach dem Trocknen wird die Folie rekonditioniert durch Anheben des Wassergehaltes auf etwa 15 Gewichtsprozent und abgelängt und verpackt.

### Beispiel 2

Zur Herstellung einer Kollagenfolie mit Hyaluronsäure wird gemäß Beispiel 1 verfahren, jedoch mit folgendem Ansatz: 45 kg der grob vorzerkleinerten Schwarten werden mit 80 kg Eis, 211 l Wasser, 1,8 l Glycerin (84 gewichtsprozentig), 0,4 l Sorbit (70 gewichtsprozentig), 2,0 kg Al₂(SO₄)3x18H₂O und 0,18 kg Hyaluronsäure (Molekulargewicht = größer als 500.000) gelöst in 1,2 l Wasser, vermischt und mit Salzsäure auf pH 2,8 eingestellt. Die weitere Verarbeitung erfolgt wie beschrieben.

## Patentansprüche

1. Wundabdeckungsmaterial in Folienform auf Basis von Kollagenfasern, dadurch gekennzeichnet, daß es im wesentlichen aus unlöslichem, teilmodifiziertem Kollagen mit folgenden Parametern
Amidstickstoff 0,18 bis 0,40 mmol/g
Glucosamin- und Galactosamingehalt: je kleiner als 5 µmol/g
Schrumpfungstemperatur 45 bis 60°C
Isoelektrischer Punkt 4,3 bis 6,0
besteht.

2. Wundabdeckungsmaterial nach Anspruch 1, dadurch gekennzeichnet, daß es, bezogen auf das Gewicht des trockenen Films 1 bis 8, vorzugsweise 2 bis 5 Gew.-% Hyaluronsäure enthält.

3. Wundabdeckungsmaterial nach Anspruch 2, dadurch gekennzeichnet, daß die Hyaluronsäure ein Molekulargewicht von größer als 500.000 aufweist.

4. Wundabdeckungsmaterial nach Anspruch 1 bis 3, dadurch gekennzeichnet, daß das Material einen Wassergehalt von etwas 12 bis 18, vorzugsweise etwa 15 Gew.-% aufweist.

5. Verfahren zur Herstellung des Wundabdeckungsmaterials nach Anspruch 1, dadurch gekennzeichnet, daß Rinderhautspalten in an sich bekannter Weise sauer gequollen, vorzerkleinert, auf Temperaturen unter 10°C gekühlt, mit physiologisch unbedendlichen Feuchthaltemitteln und ggf. mit einem physiologisch unbedenklichen organischen oder anorganischen Gerbstoff versetzt, in an sich bekannter Weise homogenisiert und zu Folien verarbeitet und der pH der extrudierten Folie auf etwa physiologisches Millieu angehoben wird.

6. Verfahren nach Anspruch 5, dadurch gekennzeichnet, daß pflanzliche oder anorganische Gerbstoffe eingesetzt werden.

7. Verfahren nach Anspruch 5 und 6, dadurch gekennzeichnet, daß Aluminiumsulfathydrat eingesetzt wird.

8. Verfahren nach Anspruch 5 bis 7, dadurch gekennzeichnet, daß die Suspension vor dem Extrudieren auf einen Trockenstoffgehalt von etwa 2 Gew.-% eingestellt wird.

9. Verfahren nach Anspruch 5 bis 8, dadurch gekennzeichnet, daß die extrudierte Folie bis zum Erreichen eines pHs von ca. 5 vor dem Trocknen mit Ammoniak begast wird.

10. Verfahren nach Anspruch 5 bis 9, dadurch gekennzeichnet, daß die Folie nach dem Trocknen auf einen Wassergehalt von etwa 12 bis 18, vorzugsweise 15 Gew.-% eingestellt wird.

11. Verfahren nach Anspruch 2 sowie Anspruch 5 bis 10, dadurch gekennzeichnet, daß der Suspension vor dem Homogenisieren 1 bis 8, vorzugsweise 2 bis 5 Gew.-% Hyaluronsäure zugegeben und kräftig durchmischt wird.

12. Wundabdeckungsmaterial nach Anspruch 1 bis 4, dadurch gekennzeichnet, daß es in an sich bekannter Weise mit Filmen oder Schäumen auf Basis eines physiologisch unbedenklichen Kunststoffes, mit schwammartigen Gebilden auf Basis von Kollagen oder Hydrokolloiden oder mit textilen Flächengebilden laminiert oder mit physiologisch unbedenklichen Metallen metallisiert ist.

## Claims

1. Foil-shaped wound covering material based on collagen fibres characterised in that it consists essentially of insoluble, partially modified collagen with the following parameters:
amidated nitrogen 0,80 to 0,30 mmol/g
glucosamine and galactosamine content: less than 5 µmol/g
shrinking temperature: from 45 to 60° centigrade
isoelectric point: 4,3 to 6,0

2. Wound covering material according to claim 1 characterised in that it contains, based on the weight of the dry film, 1 to 8, preferably 2 to 5% by weight hyaluronic acid.

3. Wound covering material according to claim 2 characterised in that the hyaluronic acid has a molecular weight of more than 500.000.

4. Wound covering material according to claims 1 to 3 characterised in that it has a water content of about 12 to 18, preferably 15% by weight.

5. Method for preparing the wound covering material according to claim 1 characterised in that skived cowhide is macerated in an acidic medium in a way known per se, comminuted, cooled to a temperature below 10° centigrade, mixed with physiologically safe humectants and, in case, physiologically safe organic or inorganic tanning agents, homogenised and extruded as films in a way known per se and that the pH of the film is raised to an about physiologic medium.

6. Method according to claim 5 characterised in the use of plant or inorganic tanning agents.

7. Method according to claim 6 characterised in use of aluminium sulfate-hydrate.

8. Method according to claims 5 to 7 characterised in that the suspension is adjusted to a dry matter content of about 2% by weight prior to extrusion.

9. Method according to claims 5 to 8 characterised in that the extruded film is treated with gaseous ammonia to a pH of about 5 prior to drying.

10. Method according to claims 5 to 9 characterised in that the film is adjusted to a water content of 12 to 18, preferably 15% by weight after drying.

11. Method according to claims 5 to 10 characterised in that the suspension prior to homogenisation is supplemented with 1 to 8, preferably 2 to 5% by weight of hyaluronic acid and mixed vigorously.

12. Wound covering material according to claims 1 to 4 characterised in that it is laminated in a way known per se with further films or foams of physiologically safe plastics, spongelike collagen or hydrocolloid based material or textile tissue or that it is metallized with physiologically safe metals.

## Revendications

1. Couverture pour blessures sous forme de film à base des fibres de collagène, caractérisée en ce qu'elle consiste essentiellement du collagène insoluble et partiellement modifié avec des paramètres suivants
nitrogène amidé: 0,18 jusqu'à 0,40 mmol/g
teneur en glucosamine et galactosamine:
plus petite que 5µmol/g
température de contraction: 45 jusqu'à 60°C
point isoélectrique: 4,3 jusqu'à 6,0

2. Couverture selon la revendication 1 caractérisée en ce qu'elle contient par rapport au poids du film sec, 1 jusqu'à 8, préférablement 2 jusqu'à 5 pourcent par rapport au poids, de l'acide hyaluronique.

3. Couverture selon la revendication 2, caractérisée en ce que l'acide hyaluronique a un poids moléculaire plus grand que 500.000.

4. Couverture selon les revendications 1 jusqu'à 3 caractérisée en ce que le matériel a une teneur en eau d'environ 12 jusqu'à 18, préférablement environ 15 pourcent par rapport au poids.

5. Méthode de produire les couvertures pour blessures selon la revendication 1 caractérisée en ce que la peau de boeuf refendue est macérée en milieu acide de manière déjà connue, préconcassée, refroidie jusqu'à dessous de 10°C, mélangée avec des humifectants physiologicalement admissible et mélangée, le cas échéant, avec des matières tannantes, homogénisée de manière déjà connue et convertie en films et que le pH du film extrudé est augmenté environ jusqu'au milieu physiologique.

6. Méthode selon la revendication 5 caractérisée en ce que les matières tannantes végétables ou inorganiques sont utilisées.

7. Méthode selon les revendications 5 et 6 caractérisée en ce que le sulfate-hydrate d'aluminium est utilisé.

8. Méthode selon les revendications 5 jusqu'à 7 caractérisée en ce que la suspension est ajustée à une teneur en matière sèche de 2 pourcent, par rapport au poids, avant l'extrusion.

9. Méthode selon les revendications 5 jusqu'à 8 caractérisée en ce que le film extrudé est traité avec du gaz ammoniac jusqu'au pH 5 environ avant le séchage.

10. Méthode selon les revendications 5 jusqu'à 9 caractérisée en ce que le film extrudé est ajusté à une teneur en eau d'environ 12 jusqu'à 18, préférablement 15 pourcent, par rapport au poids, après le séchage.

11. Méthode selon les revendications 2 et 5 jusqu'à 10 caractérisée en ce que la suspension est mélangée avec 1 jusqu'à 8, préférablement 2 jusqu'à 5 pourcent par rapport au poids, de l'acide hyaluronique et agitée vigoureusement.

12. Couverture selon les revendications 1 jusqu'à 4 caractérisée en ce qu'elle est laminée d'une manière déjà connue avec des films ou mousses à base des matières synthétiques physiologicalement admissibles, avec des éponges à base de collagène ou des hydrocolloides ou avec des produits plans textiles ou est métallisée avec des métaux physiologicalement admissibles.
